# EUROPEAN PATENT APPLICATION

(11) **EP 2 417 953 A1**
(43) Date of publication of application: **15.02.2012**
(21) Application number: 10755874.4
(22) Date of filing: 10.03.2010
(51) Int. Cl.: A61F 13/20

(54) **TAMPON**

(30) Priority: 23.03.2009 JP 2009070648
(71) Applicant: Uni-Charm Corporation, Ehime 799-0111 (JP)
(72) Inventor: WADA, Mitsuhiro, Kanonji-shi Kagawa 7691602 (JP); NOZAKI, Satoshi, Kanonji-shi Kagawa 769-1602 (JP)
(74) Representative: Vaughan, Jennifer Ann
(86) International application number: PCT/JP2010/054036
(87) International publication number: WO 2010/110074

(57) **Abstract**

A tampon has an absorbent body that absorbs liquid. The absorbent body includes, on its outer surface, an applied portion to which an agent is applied. The agent is a mixture of an active pharmaceutical ingredient, a first water-soluble carrier that carries the active pharmaceutical ingredient and is a main ingredient of the agent, and a second water-soluble carrier that carries the active pharmaceutical ingredient and has a melting point lower than that of the first water-soluble carrier.

## Description

### Technical Field

The invention relates to a tampon.

### Background Art

Tampons including an absorbent body that absorbs liquid such as menstrual blood are well known. In some of such tampons, an agent is applied onto an outer surface of the absorbent body thereof.

### Citation List

### [Patent Literature]

[PTL 1] Japanese Unexamined Patent Application Publication (Translation of PCT Application) No. 2005-536237

### Summary of the Invention

### Technical Problem

There was a possibility that the agent does not properly take effect while the foregoing tampon to which the agent is applied is inserted in the vaginal cavity. Therefore, a tampon having an agent that properly takes effect has been demanded.

This invention has been made in view of the above problems, and an advantage thereof is to provide a tampon having an agent that properly takes effect while the tampon is inserted in the vaginal cavity.

### Solution to Problem

An aspect of the invention to achieve the above advantage is a tampon having an absorbent body that absorbs liquid, including:
on an outer surface of the absorbent body,
   an applied portion to which an agent is applied,
   the agent being a mixture of
   an active pharmaceutical ingredient,
   a first water-soluble carrier that carries the active pharmaceutical ingredient and is a main ingredient of the agent, and
   a second water-soluble carrier that carries the active pharmaceutical ingredient and has a melting point lower than that of the first water-soluble carrier.

Other features of this invention will become apparent from the description in this specification and the attached drawings.

### Effects of the Invention

According to the invention, a tampon having an agent that properly takes effect while the tampon is inserted in the vaginal cavity is achieved.

### BRIEF DESCRIPTION OF THE DRAWINGS

FIG. 1 is a cross-sectional view showing components of a tampon 10.
FIG. 2 is a cross-sectional view showing the components of the tampon 10.
FIG. 3 is an external view of a tampon body 20.
FIG. 4 is an external view of an outer tube 40.
FIG. 5 is a view of the outer tube 40 shown in FIG. 4 from its front end.
FIG. 6 is a cross-sectional view take along line A-A in FIGS. 1 or 2.
FIG. 7 is a magnified view of FIG. 2.
FIG. 8 is a flowchart showing the production flow of the tampon body 20.
FIGS. 9A to 9D are schematic diagrams showing transition of the tampon body 20 to a finished product.
FIG. 10 is a schematic diagram showing a section of the manufacturing apparatus 100 for the tampon 10, which manufactures the tampon body 20.
FIG. 11 is a schematic diagram of the manufacturing apparatus 100 shown in FIG. 10 viewed from above.
FIG. 12 is a diagram showing a pattern according to the other embodiment.
FIG. 13 is a diagram showing a pattern according to the other embodiment.

### Mode for Carrying Out the Invention

At least the following matters will be made clear by the description in the present specification and the accompanying drawings.

A tampon having an absorbent body that absorbs liquid, including:
on an outer surface of the absorbent body,
   an applied portion to which an agent is applied,
   the agent being a mixture of
   an active pharmaceutical ingredient,
   a first water-soluble carrier that carries the active pharmaceutical ingredient and is a main ingredient of the agent, and
   a second water-soluble carrier that carries the active pharmaceutical ingredient and has a melting point lower than that of the first water-soluble carrier.
In such a case, a tampon having an agent that properly takes effect while the tampon being within the vaginal cavity is achieved.

In such a tampon, desirably
the absorbent body includes, on the outer surface thereof, the applied portion to which the agent is applied and a non-applied portion to which the agent is not applied, and
a pattern is formed by the applied portion and the non-applied portion on the outer surface.
This can definitely make the tampon appear attractive.

In such a tampon, desirably
the first water-soluble carrier has a melting point higher than body temperature,
the second water-soluble carrier has a melting point lower than or equal to body temperature.
In such a case, a tampon having an agent that properly takes effect while the tampon is inserted in the vaginal cavity is achieved more definitely.

### === Configuration of Tampon ===

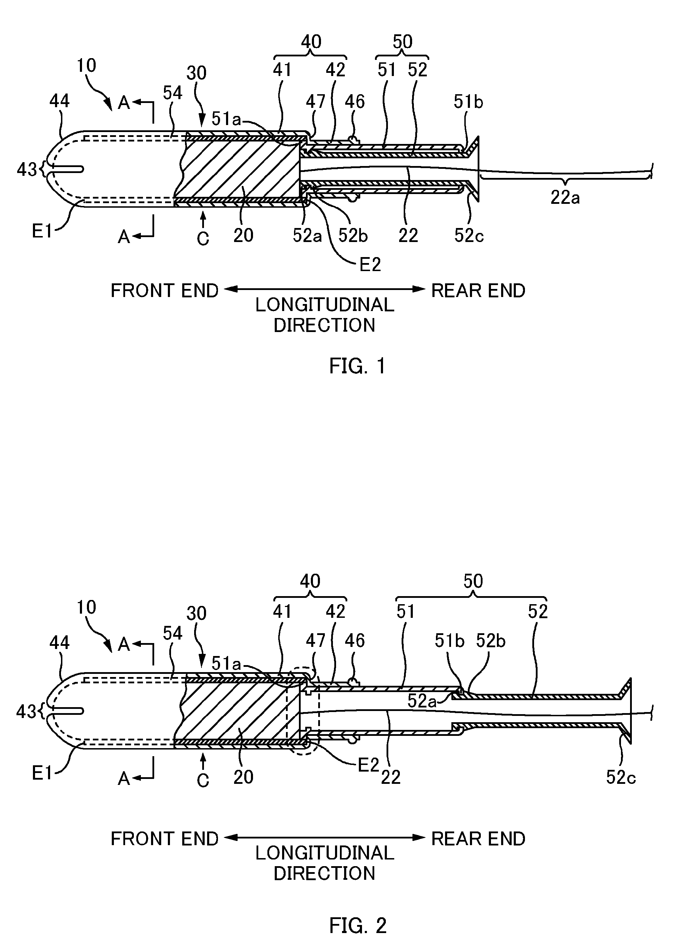

Firstly, the configuration of a tampon 10 will be described with reference to FIGS. 1 to 5.

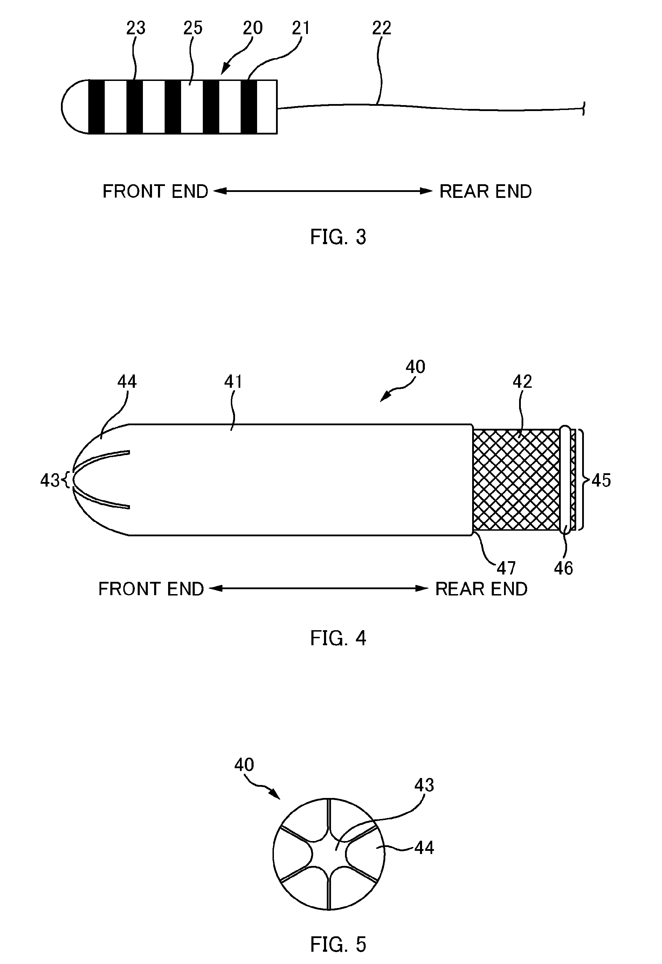

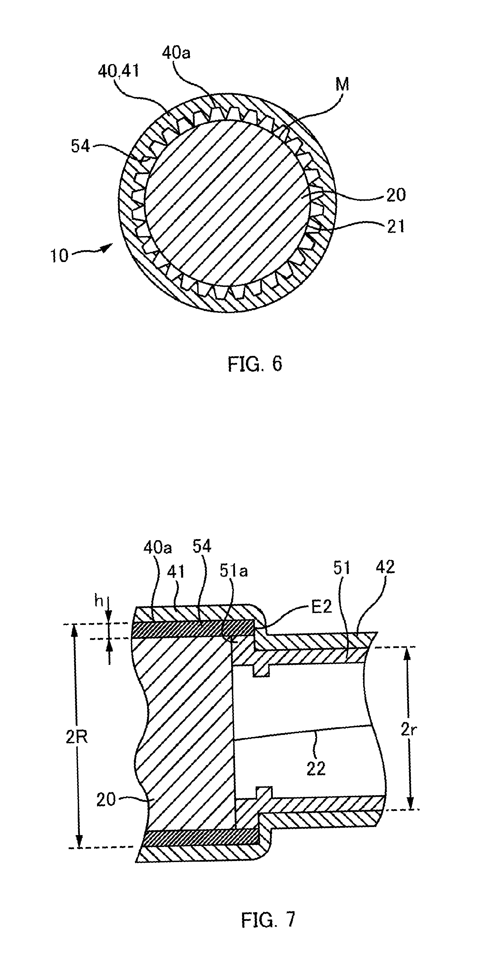

FIGS. 1 and 2 are cross-sectional views showing components of the tampon 10. FIG. 1 shows the tampon 10 with its inner tube 50 shortened, and FIG. 2 shows the tampon 10 with its inner tube 50 elongated. FIG. 3 is an external view of a tampon body 20. FIG. 4 is an external view of an outer tube 40. FIG. 5 is a view of the outer tube 40 shown in FIG. 4 from its front end. FIG. 6 is a cross-sectional view taken along A-A of FIGS. 1 or 2. FIG. 7 is a magnified view of FIG. 2. In the following description, an end inserted into the vaginal cavity in a longitudinal direction of the tampon 10 is referred to as a front end and the opposite end is referred to as a rear end.

As shown in FIGS. 1 and 2, the tampon 10 of the present embodiment includes the tampon body 20 as an example of the absorbent body, and an applicator 30 having the outer tube 40 as an example of housing cylinder and the inner tube 50 as an example of a pushing member.

The tampon body 20 is a thing to fill the vaginal cavity and absorb liquid such as menstrual blood. This tampon body 20 is formed by covering an absorbent main body (wool-like body) made of rayon fiber, with a cover made of polyester spunbond nonwoven fabric. The tampon body 20 is shaped substantially like a bullet.

Further, an agent M is adhered on an outer surface 21 of the tampon body 20. This agent M is a light brown colorant (a colored substance), and is a mixture of a pine bark extract (flavangenol (R) manufactured by Toyo Shinyaku Co., Ltd.) and a polyethylene glycol, the pine bark extract being as an example of an active pharmaceutical ingredient that is administered to the inside of the vaginal cavity and performs antioxidant activity, anti-inflammatory activity, antibiotic activity, antiviral activity, antiallergic activity, deodorization, vasodilation, inhibitory action on lipid peroxidation etc, and the polyethylene glycol being as an example of a water-soluble carrier that carries the pine bark extract.

More specifically, the agent M according to the present embodiment includes, as water-soluble carrier (polyethylene glycol), two water-soluble carriers whose melting points (in other words, freezing point) are different from each other. In other words, the agent M includes: a polyethylene glycol with a molecular weight of 1540 (hereinafter referred to as a first polyethylene glycol) as an example of a first water-soluble carrier, and a polyethylene glycol with a molecular weight of 1000 (hereinafter referred to as a second polyethylene glycol) as an example of a second water-soluble carrier. The melting point (freezing point) of the first polyethylene glycol is approximately 45°C, higher than body temperature (37°C). On the other hand, the melting point (freezing point) of the second polyethylene glycol is approximately 37°C, which is lower than the melting point of the first polyethylene glycol and is lower than or equal to body temperature. As mentioned above, the agent M is a mixture of the pine bark extract, the first polyethylene glycol, and the second polyethylene glycol.

In the present embodiment, the mixture ratio of the pine bark extract, the first polyethylene glycol, and the second polyethylene glycol is 1:3:1 (that is, the pine bark extract has 20 weight percent, the first polyethylene glycol 60 weight percent, and the second polyethylene glycol 20 weight percent). That is, the first polyethylene glycol is a main ingredient of the agent M.

Further, the tampon body 20 according to the present embodiment, as shown in FIG. 3, has a pattern on the outer surface 21. In other words, the tampon body 20 includes on its outer surface 21 the applied portions 23 to which the agent M is applied and non-applied portions 25 to which the agent M is not applied. Also, the pattern is formed on the outer surface 21 by the applied portions 23 and non-applied portions 25 (that is, by the difference in color between the applied portions 23 and non-applied portions 25). The pattern according to the present embodiment is one that is formed by arranging alternately regularly the applied portions 23 and non-applied portions 25. Specifically, the pattern, as shown in FIG. 3, is one that is composed of rings (4 mm in width) lined up in the longitudinal direction of the tampon body 20.

Onto the tampon body 20 according to the present embodiment, a withdrawal string 22 as an example of a string is stitched. This withdrawal string 22 is a cotton string. The withdrawal string 22 extends from the rear end of the tampon body 20, and is held by a user of the tampon while attempting to remove the tampon body 20 out of the vaginal cavity. Further, as shown in FIGS. 1 and 2, the withdrawal string 22 passes inside the applicator 30 and extends somewhat beyond the rear end of the applicator 30 (the inner tube 50). That is, a part of the withdrawal string 22 is exposed outside from the rear end of the applicator 30 (the inner tube 50).

In the present embodiment, while the agent M is applied to the tampon body 20 (the applied portions 23 are included), the agent M is not applied to an exposed portion 22a of the withdrawal string 22 (the applied portions 23 are not included). Further, the withdrawal string 22 does not include any of the applied portions 23 (There is no applied portion 23 on the withdrawal string 22).

The applicator 30 is an assisting tool in order to facilitate insertion of the tampon body 20 into the vaginal cavity. The applicator 30 includes the outer tube 40 and the inner tube 50, as shown in FIGS. 1 and 2.

The outer tube 40 is for housing the tampon body 20. The outer tube 40 is a cylinder that is injection-molded from thermoplastic resin (in the present embodiment, polyethylene resin), and has suitable flexibility. The outer tube 40 has a transparency that allows the tampon body 20 housed in the outer tube to be seen from outside (in other words, the pattern formed on the tampon body 20) (for example, haze value of 90% or less; in the present embodiment, 47%). The outer tube 40 may or may not be colored; in the present embodiment, an entire surface of the outer tube 40 is colored light pink.

Further, the outer tube 40 includes: a radially-large portion 41 positioned on the front end side (in other words, the one end side in the longitudinal direction of the outer tube 40), and a radially-small portion 42 that has an internal diameter smaller than that of the radially-large portion 41 and is positioned on the rear end side, opposite the front end side (in other words, the other end side in the longitudinal direction of the outer tube 40) (the radially-large portion 41 is also larger than the radially-small portion 42 in external diameter). The front end section of the outer tube 40 is larger than the rear end thereof in external diameter (internal diameter). Thereby, an annular shoulder 47 is formed between the radially-large portion 41 and radially-small portion 42.

The radially-large portion 41 is a portion of the outer tube 40 and has a function mainly to house the tampon body 20 inside thereof. Indeed, in the tampon 10 according to the present embodiment, the tampon body 20 is housed in the radially-large portion 41 only of the radially-large portion 41 and radially-small portion 42 (therefore, in the longitudinal direction of the outer tube 40, the length of the radially-large portion 41 is larger than that of the tampon body 20). The radially-large portion 41 is a portion that is inserted into the vaginal cavity with housing the tampon body 20 therein when the tampon 10 is used.

Further, the radially-large portion 41 (the outer tube 40) includes an opening (hereinafter referred to as a front-end opening 43) on its front end, and also includes a plurality of petal portions 44 surrounding the front-end opening 43 (in the present embodiment, 6). Each of the plurality of petal portions 44 is bent in the form of an arc radially inwardly of the outer tube 40, as shown in FIG. 4. Therefore, when inserting the outer tube 40 into the vaginal cavity, the front end section of the outer tube 40 is substantially hemisphere in shape as shown in FIGS. 1 and 2, and the front-end opening 43 is substantially closed as shown in FIG. 5. When the tampon body 20 is expelled from the front-end opening 43 by the inner tube 50 described below, the front-end opening 43 opens.

The radially-small portion 42 is a section that provides a space in which of the outer tube 40 the following inner tube 50 mainly moves (However, of course, the inner tube 50 moves not only inside the radially-small portion 42 but also inside the radially-large portion 41). The radially-small portion 42 is a portion that is held by a user when the tampon 10 is used.

Further, the radially-small portion 42 (the outer tube 40) includes an opening (hereinafter referred to as a rear-end opening 45) on the rear end as shown in FIG. 4, and also includes an annular rib 46 that is formed slightly closer to the front end than the rear-end opening 45 is.

As shown in FIGS. 1, 2, 6, and 7, the outer tube 40 includes ribs (hereinafter referred to as a longitudinal ribs 54) on an inner surface 40a thereof (of the outer tube 40) along the longitudinal direction of the outer tube 40. The outer tube 40 according to the present embodiment includes the longitudinal ribs 54 in at least an area that is closer to the front end from a center C in the longitudinal direction of the outer tube 40 (see FIG. 1). Also, in the present embodiment, the outer tube 40 includes the longitudinal ribs 54, on only the radially-large portion 41 of the radially-large portion 41 and the radially-small portion 42.

As shown in FIG. 6, 32 of the longitudinal direction ribs 54 are arranged such that the ribs are equally spaced along the inner circumferential direction of the inner surface 40a. In other words, the outer tube 40 (radially-large portion 41) has three or more longitudinal direction ribs 54 that is arranged such that the ribs 54 are equally spaced along the inner circumferential direction of the inner surface 40a. In the present embodiment, the longitudinal direction ribs 54 that are adjacent to each other are not in contact with each other (do not abut and touch each other).

Each of the longitudinal direction ribs 54 is formed straight from the front end of the radially-large portion 41 to the rear end thereof, as shown in FIGS. 1 and 2. More specifically, the longitudinal direction ribs 54 according to the present embodiment are disposed of the radially-large portion 41 up to an rearmost end E2 thereof, but do not reach an foremost end of the radially-large portion 41. In other words, the petal portions 44 do not have the longitudinal rib 54, and the ribs 54 are disposed of the radially-large portion 41 up to an foremost end E1 thereof except for the petal portions.

Further, as shown in FIG. 6, the longitudinal rib 54 according to the present embodiment is a rib extending radially of the radially-large portion 41, and the width of the rib narrows as it gets closer radially to the center. As shown in FIG. 7, the height h of the longitudinal rib 54 extending radially of the radially-large portion 41 is smaller than the difference of the internal diameters of the radially-large portion 41 and radially-small portion 42 (internal diameter of radially-large portion 41 R - internal diameter of radially-small portion r = R-r) (h < R-r). In other words, the internal diameter R-h of the radially-large portion 41 considering the longitudinal ribs 54 (hereinafter referred to as, for convenience, a rib-considered internal diameter) is larger than the internal diameter r of the radially-small portion (r < R-h).

The tampon body 20 has an external diameter substantially same as rib-considered internal diameter, and the tampon body 20 is housed in the radially-large portion 41 of the outer tube 40, with being in contact only with the longitudinal rib 54 of the inner surface 40a and the longitudinal rib 54, as shown in FIG. 6. In other words, the outer surface 21 of the tampon body 20 is not in contact with the inner surface 40a, and is in contact only with a radially-extending front end section of the longitudinal ribs 54.

The inner tube 50 is for expelling the tampon body 20 from the front-end opening 43 outside the outer tube 40 by moving in the outer tube 40. This inner tube 50 is inserted into the outer tube 40, and is positioned closer to the rear end in the outer tube 40 than the tampon body 20 is. The inner tube 50 moves along the longitudinal direction of the outer tube 40 and pushes the tampon body 20 towards the front-end opening 43 from the rear. Thereby, the tampon body 20 pushes aside each of the plurality of petal portions 44 radially outwardly of the outer tube 40 (in other words, opens the front-end opening 43) and is expelled from the outer tube 40. As mentioned above, the inner tube 50 has a function to expel the tampon body 20 out of the outer tube 40 by moving the outer tube 40.

Further, the inner tube 50 according to the present embodiment has a retractable configuration in order to make the tampon 10 compact in size. Specifically, as shown in FIG. 1, when the inner tube 50 is shortened, the inner tube 50 is shorter in length than the outer tube 40 so that the length of the tampon 10 is suitable for carrying. On the other hand, as shown in FIG. 2, when the inner tube 50 is elongated, the length of the inner tube 50 is sufficient to expel the tampon body 20 outside the outer tube 40. As mentioned above, in order to make the inner tube 50 retractable, in the present embodiment, the inner tube 50 has a dual structure. Specifically, as shown in FIGS. 1 and 2, the inner tube 50 of the present embodiment includes a first inner tube 51, and a second inner tube 52 that is slidably inserted into the first inner tube 51.

The first inner tube 51 is a cylinder that is injection-molded from plastic. The first inner tube 51 has an external diameter that is slightly smaller than the internal diameter of the radially-small portion 42 of the outer tube 40. Also, the first inner tube 51 is slidably inserted into the radially-small portion 42, as shown in FIG. 1. On the outer circumferential face of the front end section of the first inner tube 51, an annular sword-guard portion 51a is formed. This sword-guard portion 51a has an external diameter that is slightly smaller than the rib-considered internal diameter of the radially-large portion 41 of the outer tube 40. Also, the sword-guard portion 51a is stopped by connecting it to an inner wall of the shoulder 47 so that the sword-guard portion 51a prevents the inner tube 50 from falling off the rear-end opening 45 of the outer tube 40. When the inner tube 50 expels the tampon body 20 out of the outer tube 40, the inner tube 50 moves such that the outer circumferential face of the sword-guard portion 51a comes into contact with the longitudinal ribs 54 of the radially-large portion 41. Further, at the rear end section of an inner circumferential face of the first inner tube 51, an annular projection 51b extending radially inwardly of the first inner tube 51 are formed, as shown in FIGS. 1 and 2.

The second inner tube 52 is a cylinder that is injection-molded from thermoplastic resin. This second inner tube 52 has an external diameter slightly smaller than the internal diameter of the first inner tube 51. The second inner tube 52 is inserted into the first inner tube 51 as shown in FIG. 1 when the inner tube 50 is shortened. The second inner tube 52 is connected to the rear end section of the first inner tube 51 at the front end section of the second inner tube 52 as shown in FIG. 2 when the inner tube 50 is elongated. On the outer circumferential face of the front end section of the second inner tube 52, are formed an arc-shaped sword-guard portion 52a and a projection section 52b that is located closer to the rear end than the sword-guard portion 52a is. The height of the projection section 52b is lower as it gets close to the rear end, as shown in FIG. 2. The space between the sword-guard portion 52a and projection section 52b of the second inner tube 52 is slightly larger than the thickness of the annular projection 51b of the first inner tube 51.

When the second inner tube 52 is pulled towards the rear end, the annular projection 51b of the first inner tube 51 is positioned between the sword-guard portion 52a and projection section 52b of the second inner tube 52. In this state, as shown in FIG. 2, the annular projection 51b is stopped by connecting to the sword-guard portion 52a and projection section 52b, and the first inner tube 51 connects to the second inner tube 52.

Further, as shown in FIGS. 1 and 2, a flared portion 52c is formed on rear end section of the second inner tube 52. The external diameter of the flared portion 52c is desirably at least larger than the internal diameter of the first inner tube 51 and larger than or equal to the internal diameter of the radially-small portion 42 of the outer tube 40.

### Effectiveness of Tampon 10 according to Present Embodiment ===

As mentioned above, in the tampon 10 according to the present embodiment, the tampon body 20 includes, on its outer surface 21, applied portions 23 to which the agent M is applied. The agent M is the mixture of: pine bark extract as an example of active pharmaceutical ingredient; first polyethylene glycol as an example of a first water-soluble carrier that carries the pine bark extract and is the main ingredient of the agent M; and the second polyethylene glycol as an example of a second water-soluble carrier that carries the pine bark extract and whose melting point is lower than that of the first polyethylene glycol. Thereby, the tampon 10 having the agent M that properly takes effect while the tampon being within the vaginal cavity is achieved.

Regarding the foregoing, the description will be made comparing the tampon 10 according to the present embodiment (the present example) and a tampon according to comparative example. Comparing the tampon 10 according to the present embodiment to the tampon according to comparative example, in both of them, the tampon body 20 includes on its outer surface 21 the applied portions 23 to which the agent M is applied, and also the agent M is the mixture of the pine bark extract and the polyethylene glycol. However, in the tampon according to comparative example, one type of polyethylene glycol alone is mixed with the pine bark extract.

Consider the case where a polyethylene glycol having a low melting point (for example, the foregoing second polyethylene glycol whose melting point is lower than or equal to body temperature) is selected. When storing a tampon in a place where the temperature is high (a warehouse, for example), the agent melts. This may cause a problem that the melted agent is absorbed into or falls off the tampon body. When using the tampon in which the foregoing absorption or falling off has happened (that is, inserting the tampon into the vaginal cavity), the amount of the agent adhering onto the outer surface decreases. Therefore, the agent cannot be transferred to the vaginal mucosa appropriately. That is, the agent does not properly take effect when the tampon is inserted into the vaginal cavity.

On the other hand, in the case where a polyethylene glycol having high melting point (for example, the foregoing first polyethylene glycol whose melting point is higher than body temperature) is selected, the agent is less likely to melt even when the tampon is inserted into the vaginal cavity. Melting of the agent is considerably delayed (melting of the agent is delayed until menstruation occurs, that is, until water such as menstrual blood etc comes out sufficiently for the polyethylene glycol to dissolve in the water; melting of the agent is less likely to happen before menstruation). In other words, the agent does not properly take effect when a tampon is inserted into vaginal cavity (this makes the agent less rapid-acting).

As mentioned above, in the tampon according to the comparative example, regardless of the melting points, a problem that the agent does not properly take effect when a tampon is inserted into the vaginal cavity may arise.

As opposed thereto, in the present example, two types of polyethylene glycols are employed to be mixed with the pine bark extract. Therefore, polyethylene glycols having two melting points can be mixed with the pine bark extract. That is, as the tampon 10 according to the present embodiment, the first polyethylene glycol having a higher melting point than body temperature and the second polyethylene glycol having a melting point lower than or equal to body temperature can be mixed with the pine bark extract. Further, the foregoing problems are properly solved by employing as the main ingredient of the agent M the polyethylene glycol having a higher melting point (in the present embodiment, the first polyethylene glycol).

That is, because the first polyethylene glycol having a higher melting point is the main ingredient of the agent M, even if storing the tampon 10 in a place where the temperature is high (a warehouse, for example) results in melting of the second polyethylene glycol, the frozen first polyethylene glycol prevents the melted second polyethylene glycol from being absorbed or falling off as mentioned above (in the agent M, the second polyethylene glycol stays in a fluid form within the first polyethylene glycol). Then, the absorption or falling off is prevented. This makes it possible to properly avoid the foregoing problem that the agent M cannot be transferred to the vaginal mucosa appropriately because the amount of the agent M adhering onto the outer surface 21 decreases.

Further, when inserting the tampon 10 into the vaginal cavity, while the first polyethylene glycol is freezing, the second polyethylene glycol is melting due to body temperature. When or after inserting the tampon 10, contact of the agent M adhering to the outer surface 21 with the vaginal mucosa creates friction; by which the melted second polyethylene glycol that is in a fluid form is pushed out of the frozen first polyethylene glycol. Therefore, even before water such as menstrual blood etc comes out sufficiently (for example, before menstruation), the agent M properly takes effect (the agent M can be more rapid-acting) because of the second polyethylene glycol and pine bark extract carried thereby. In such a state as menstruation in which water such as menstrual blood etc comes out sufficiently and the first polyethylene glycol dissolves in the water, the agent M properly takes effect because of the first polyethylene glycol and the pine bark extract carried thereby. That is, this enables the agent M to take effect gradually and ideally.

As mentioned above, according to the present embodiment, the tampon 10 having the agent M that properly takes effect while the tampon is inserted in the vaginal cavity is achieved.

Further, in the present embodiment, the tampon body 20 includes the applied portions 23 to which the agent M is applied and the non-applied portions 25 to which the agent is not applied on the outer surface 21 of the body 20. Also, by the applied portions 23 and the non-applied portions 25, the pattern is formed on the outer surface 21. This makes appearance of the tampon body 20 good, and can make the tampon 10 appear attractive (this effect leads to make it less reluctant to insert the tampon 10). Further, the agent M is mixture of: the pine bark extract as an example of active pharmaceutical ingredient; the first polyethylene glycol as an example of a first water-soluble carrier that carries the pine bark extract and is the main ingredient of the agent M; and the second polyethylene glycol as an example of a second water-soluble carrier that carries the pine bark extract and whose melting point is lower than that of the first polyethylene glycol. Therefore, as mentioned above, the problem that the agent M falls off the tampon body 20 when the tampon 10 is stored is less likely to arise. As a result, in the tampon 10 according to the present embodiment, the pattern is less likely to distort and it can definitely make the tampon 10 appear attractive.

Further, in the foregoing embodiment, the first water-soluble carrier (the first polyethylene glycol) has a higher melting point than body temperature, and the second water-soluble carrier (the second polyethylene glycol) has a melting point lower than and equal to body temperature. Therefore, the tampon 10 having the agent M that properly takes effect while the tampon is inserted in the vaginal cavity is achieved more definitely.

### === Manufacturing Method for Tampon 10 ===

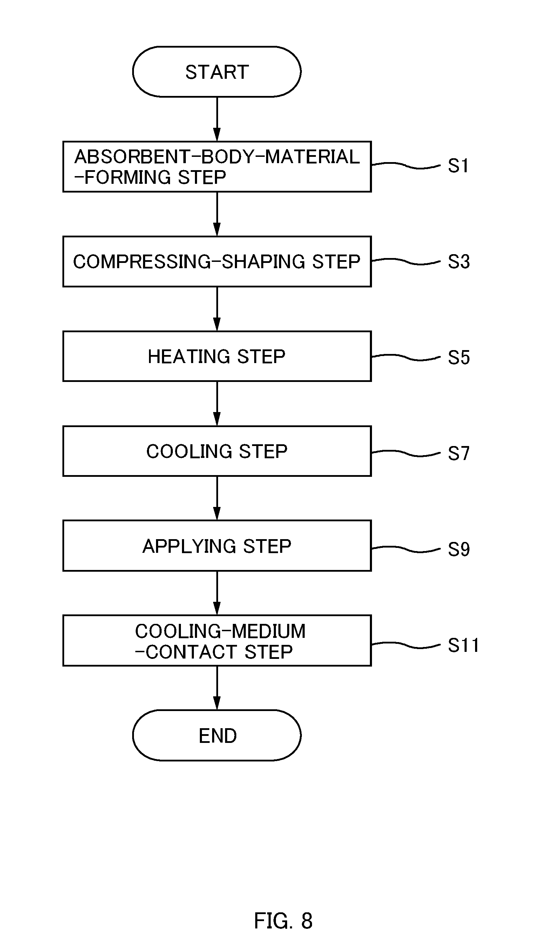

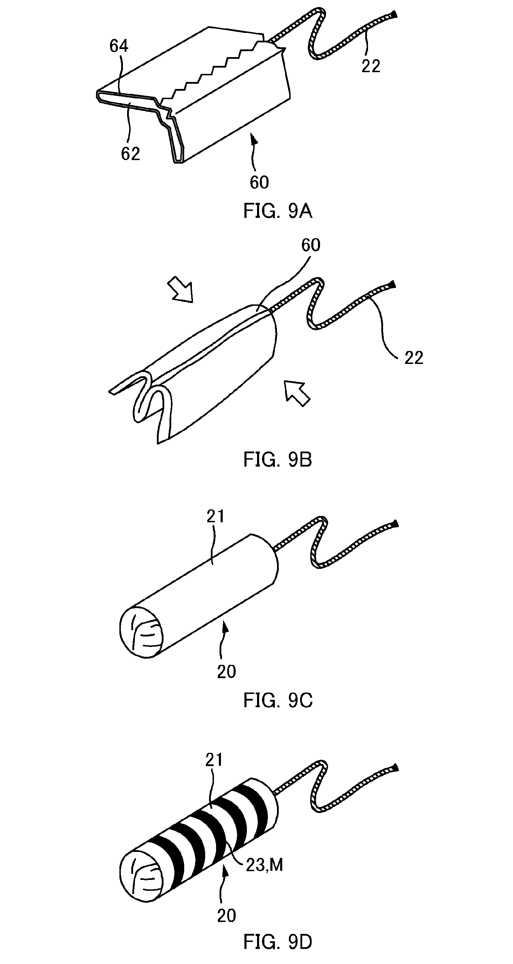

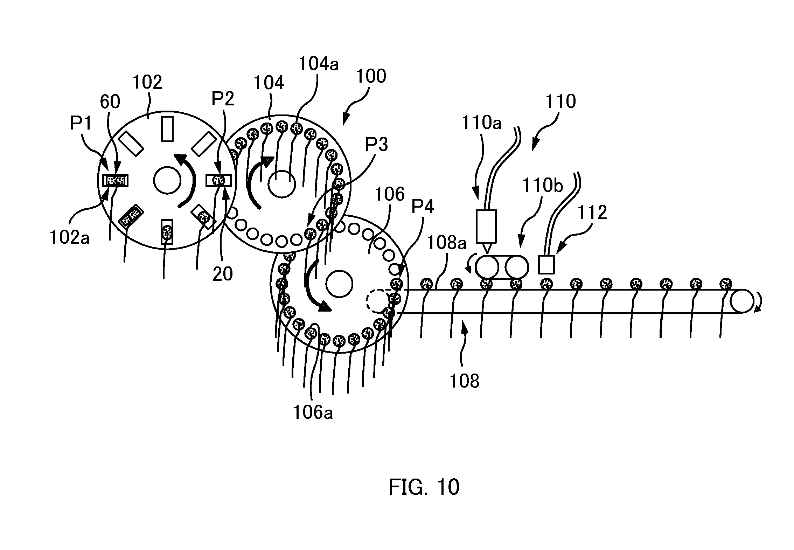

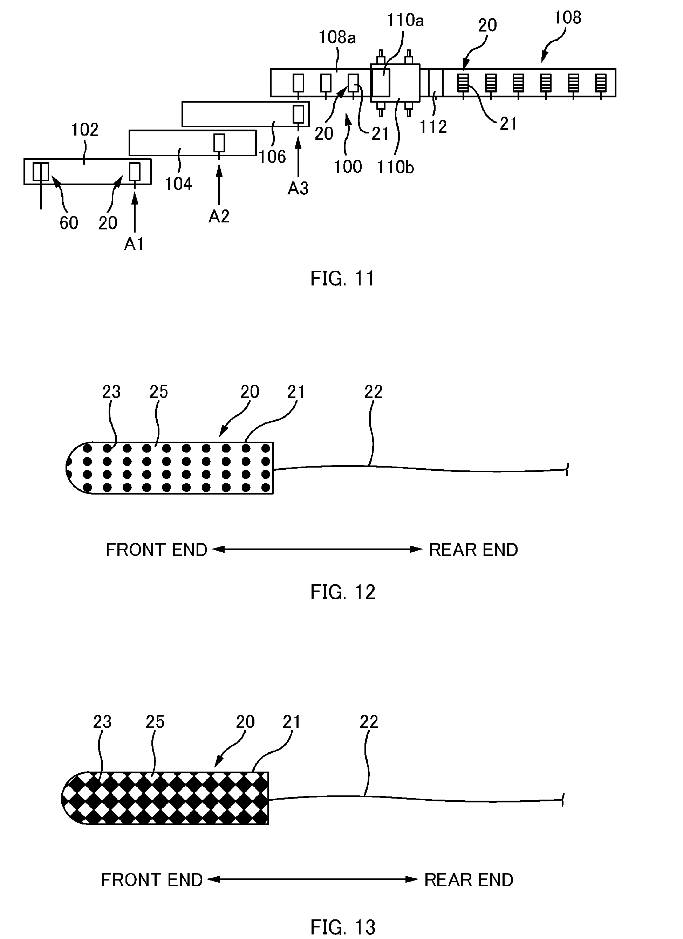

Next, a manufacturing method for manufacturing the foregoing tampon 10 will be described with reference to FIGS. 8 to 11. FIG. 8 is a flowchart showing the production flow of the tampon body 20. FIGS. 9A to 9D are schematic diagrams showing the transition of the tampon body 20 to a finished product. FIG. 10 is a schematic diagram showing a section of the manufacturing apparatus 100 for the tampon 10, which manufactures the tampon body 20. FIG. 11 is a schematic diagram of the manufacturing apparatus 100 shown in FIG. 10 viewed from above.

The manufacturing process of the tampon 10 is divided into: a process in which the components of the tampon 10 (that is, the tampon body 20, the outer tube 40, the first inner tube 51, and the second inner tube 52) are manufactured, and a process in which these components are assembled. This section will describe the process in which the tampon body 20 (more precisely, the tampon body 20 having the withdrawal string 22) is manufactured.

The production flow of FIG. 8 starts with an absorbent-body-material-forming step (step S1). In this step, firstly, the absorbent main body 62 (wool-like body) is covered with a cover 64 (wrapped with the cover 64). Then, the absorbent main body 62 covered with the cover 64 is cut into a predetermined shape and size. Thereby, an absorbent-body material 60 is formed (that is, the base material of the tampon body 20). in this step, the absorbent-body material 60 undergoes a process in which the withdrawal string 22 is stitched to the absorbent-body material 60 (FIG. 9A shows a state of the absorbent-body material 60 after the step is finished).

Next, the tampon body 20 is obtained by compressing and shaping the absorbent-body material 60 (compressing-shaping step of step S3).

FIGS. 10 and 11 show a compressing-shaping drum 102 as an example of a compression-shaping unit; the compressing-shaping drum 102 has a function to compress and shape the absorbent-body material 60 (also, the tampon body 20 is obtained thereby). Indeed, the compressing-shaping drum 102 is a drum-shaped rotatable unit, and includes a plurality of holding sections 102a (in the present embodiment, 8) positioned radially. The absorbent-body material 60 is inserted successively into the holding section 102a at a first position P1 (FIG. 10), and the inserted absorbent-body material 60 rotates and moves to a second position P2 (FIG. 10) in conjunction with rotation of the compressing-shaping drum 102. Then, while rotating and moving, the absorbent-body material 60 is compressed from the both side thereof in the holding section 102a (FIG. 9B shows a state of the absorbent-body material 60 being compressed).

Next, by heating the tampon body 20 that is obtained by compressing and shaping the absorbent-body material 60 with the compressing-shaping drum 102, the shape of the tampon body 20 is fixed (heating step of step S5).

FIGS. 10 and 11 show a heating drum 104 as an example of a heating unit; the heating drum 104 has a function to heat the tampon body 20 that is obtained by compressing and shaping the absorbent-body material 60 with the compressing-shaping drum 102 (further, thereby the shape of the tampon body 20 is fixed). Indeed, the heating drum 104 is a drum-shaped rotatable unit whose temperature is controlled at 110 degree, for example (a temperature between 100 and 180 degree is preferable). The drum 104 includes many of holding sections 104a radially positioned. The tampon body 20 is transferred successively at a second position P2 (FIG. 10) from the holding section 102a of the compressing-shaping drum 102 to the holding section 104a of the heating drum 104, by the pushing of a pusher (not shown) (the direction in which the pusher pushes the body is shown with an arrow A1 in FIG. 11). Then, the tampon body 20 that is transferred to the holding section 104a rotates and moves to the third position P3 (FIG. 10) in conjunction with rotation of the heating drum 104. While rotating and moving, the tampon body 20 is heated in the holding section 104a and the shape of the tampon body 20 is fixed. The holding section 104a is a hole having a shape corresponding to the shape of the tampon body 20 (the tampon body 20 fits in the hole). Also, the heat of the heating drum 104 is effectively conducted to the tampon body 20. Further, at the same time when transferring the tampon body 20 from the compressing-shaping drum 102 to the heating drum 104, the tampon body 20 undergoes a process in which the front end is formed in the shape of a bullet. FIG. 9C shows a state of the tampon body 20 after the heating step is finished.

Next, the tampon body 20 whose shape is fixed by the heating drum 104 is cooled (cooling step of step S7).

FIGS. 10 and 11 show a cooling drum 106 as an example of a cooling unit; the cooling drum 106 has a function to cool the tampon body 20 whose shape is fixed by the heating drum 104. Indeed, the cooling drum 106 is a drum-shaped rotatable unit whose temperature is controlled at 25°C for example, and includes many of holding sections 106a positioned radially. The tampon body 20 is transferred successively at a third position P3 (FIG. 10) from the holding section 104a of the heating drum 104 to the holding section 106a of the cooling drum 106 by the pushing of a pusher (not shown) (the direction in which the pusher pushes the body is shown with an arrow A2 in FIG. 11). Then, the tampon body 20 that is transferred to the holding section 106a rotates and moves to a fourth position P4 (FIG. 10) in conjunction with rotation of the cooling drum 106. While rotating and moving, the tampon body 20 is cooled in the holding section 106a. In similar to the holding section 104a, the holding section 106a is a hole having a shape corresponding to the tampon body 20 (the tampon body 20 fits in the hole), and is configured such that the tampon body 20 is cooled effectively by the cooling drum 106.

The tampon body 20 that has rotated and moved to the fourth position P4 (FIG. 10) is transferred successively at the fourth position P4 from the holding section 106a of the cooling drum 106 to a conveyor unit 108 (specifically, a conveyor belt 108a disposed of the conveyor unit 108) by the pushing of a pusher (not shown) (the direction in which the pusher pushes the body is shown with an arrow A3 in FIG. 11). The conveyor belt 108a is an endless conveyor belt for the tampon body; the conveyor belt 108a holds the tampon body 20 such that the longitudinal direction of the tampon body 20 is aligned in the width direction of the conveyor belt 108a. The conveyor belt 108a conveys the tampon body 20 by rotating and moving. The temperature of the conveyor belt 108a is controlled, for example, at 25°C, the conveyor belt 108a also has a function as a cooling unit that cools the tampon body 20. Indeed, the tampon body 20 whose shape is fixed by the heating drum 104 is cooled by the cooling unit, first in the cooling drum 106 and second in the conveyor belt 108a.

Next, melted agent M is applied to the outer surface 21 of the tampon body 20 (applying step of step S9)
FIGS. 10 and 11 show a applying unit 110, which has a function to apply melted agent M onto the outer surface 21 of the tampon body 20. The applying unit 110 includes a supplying unit 110a and a transferring belt 110b.

The supplying unit 110a is for supplying the melted agent M to the transferring belt 110b. In the present embodiment, the supplying unit 110a melts the agent M and applies the melted agent M to the transferring belt 110b.

The transferring belt 110b is for transferring and applying the melted agent M onto the outer surface 21 while the belt being in contact with the outer surface 21 of the tampon body 20. In the present embodiment, the transferring belt 110b is an endless conveyor belt for an agent, which conveys the agent M by rotating and moving with holding the agent M applied by the supplying unit 110a. Then the conveyed agent M reaches a contact position at which the transferring belt 110b comes into contact with the outer surface 21 of the tampon body 20 being conveyed by the conveyor belt 108a, and the agent M is transferred and applied to the outer surface 21.

As shown in FIG. 10, when the agent M is applied to the outer surface 21, the tampon body 20 is being sandwiched between the transferring belt 110b and the conveyor belt 108a. In the present embodiment, the speeds in the direction from left to right in FIG. 10 (hereinafter referred to as merely a left-to-right direction) are controlled such that the speed of the transferring belt 110b is greater than that of the conveyor belt 108a. Therefore, while the tampon body 20 is sandwiched between the transferring belt 110b and the conveyor belt 108a, the tampon body 20 rotates on the conveyor belt 108a and moves in the left-to-right direction. Therefore (because of the rotation), the agent M is applied onto the entire outer surface 21 of the tampon body 20 circumference-wise.

As mentioned above, the tampon body 20 is cooled by the cooling unit whose temperature is controlled at 25°C first by the cooling drum 106 and second by the conveyor belt 108a. Therefore, the applying unit 110 applies the melted agent M onto the outer surface 21 of the tampon body 20, the outer surface being cooled to approximately 25°C by the cooling unit. Because, the melting point (freezing point) of the main ingredient of the agent M (that is, the first polyethylene glycol) is approximately 45°C as mentioned above, the melted agent M freezes rapidly (instantly) when applying the agent M onto the outer surface 21 of the tampon body 20. As mentioned above, in the cooling step of step S7, the cooling unit cools the tampon body 20 whose shape is fixed by the heating drum 104 so that a temperature of its outer surface 21 becomes a temperature lower than or equal to the freezing point of the main ingredient of the agent M. In the applying step of step S9, the applying unit 110 applies the melted agent M onto the outer surface 21 of the tampon body 20, the outer surface 21 having a temperature lower than or equal to the freezing point (that is, cooled to a temperature lower than or equal to the freezing point).

Next, a cooling medium is brought into contact with the agent M applied to the outer surface 21, the cooling medium being cooled to a temperature lower than or equal to the freezing point of the main ingredient of the agent M (in the present embodiment, approximately 45°C) (cooling-medium-contact step of step S11).

10 and 11 show a cool-air-blowing unit 112 as an example of a cooling-medium-contact unit that brings the cooling medium into contact; the cool-air-blowing unit 112 has a function to bring cool air as a cooling medium into contact with the agent M that is applied by the applying unit 110 onto the outer surface 21, the cool air being cooled to a temperature lower than or equal to the freezing point of the main ingredient of the agent M. That is, the cool-air-blowing unit 112 brings the cooling medium into contact with the agent M by blowing cool air onto the agent M, the cool air being cooled to a temperature lower than or equal to 45°C (in the present embodiment, 25°C).

Further, as mentioned above, the temperature of the conveyor belt 108a is controlled at 25°C, the conveyor belt 108a is cooled, and the tampon body 20 rotates on the conveyor belt 108a with being sandwiched between the transferring belt 110b and the conveyor belt 108a. As a result, the agent M applied to the outer surface 21 comes instantly into contact with the conveyor belt 108a. Therefore, the conveyor belt 108a has a function as a cooling medium that is cooled to a temperature lower than or equal to the freezing point. In other words, the conveyor unit 108 has a function as a cooling-medium-contact unit that brings the conveyor belt 108a as a cooling medium into contact with the agent M that is applied onto the outer surface 21, the conveyor belt being cooled to a temperature lower than or equal to the freezing point.

As mentioned above, in the present embodiment, the melted agent M is applied onto the outer surface 21 of the tampon body 20 cooled to a temperature lower than or equal to the freezing point. In addition thereto, the agent M applied on the outer surface 21 is brought into contact with the cooling medium cooled to a temperature lower than or equal to the freezing point. Therefore, when the melted agent M is applied to the outer surface 21 of the tampon body 20, the agent M freezes more rapidly. Then, the cooling-medium-contact step is performed, and the manufacturing process of the tampon body 20 is finished. FIG. 9D shows a state of the tampon body 20 after the cooling-medium-contact step is finished.

### === Other Embodiments ===

Above, based on the above embodiments, the tampon according to the invention is described, but the above embodiments of the invention are for facilitating understanding of the invention, and are not limiting of the invention. The invention can of course be altered and improved without departing from the gist thereof, and equivalents are intended to be embraced therein.

Further, in the foregoing embodiment, the tampon 10 having the applicator 30 is provided as an example of a tampon, but the invention is not limited thereto. A tampon without an applicator can be employed.

Further, in the foregoing embodiment, a pattern that is composed of rings lined up in the longitudinal direction of the tampon body 20 is provided as an example of a pattern, but the invention is not limited thereto. For example, a spotted pattern shown in FIG. 12 or a checkerboard pattern shown in FIG. 13 also can be employed. FIGS. 12 and 13 correspond to FIG. 3 and are diagrams showing a pattern according to the other embodiment.

Further, in the foregoing embodiment, the pine bark extract is provided as an example of an active pharmaceutical ingredient of the agent M, but the invention is not limited thereto. For example, a plant extract such as red clover, polygonum indigo extract, indirubin or the like can be employed. In addition thereto, flavangenol ® is provided as an example of a pine bark extract, but the invention is not limited thereto. For example, pycnogenol ® which Nihon SiberHegner K.K deals in or enzogenol ® which Valentine Company Limited deals in can be employed.

Further, in the foregoing embodiment, polyethylene glycol with a molecular weight of 1000 (with melting point at approximately 37°C) is provided as an example of the second water-soluble carrier, but the invention is not limited thereto. For example, polyethylene glycol with a molecular weight of 600 (with melting point at approximately 20°C) can be employed.

The polyethylene glycol with molecular weight of 1000 is difficult to melt when the tampon 10 is stored, and is superior because this makes it possible to properly prevent the polyethylene glycol from absorption or falling off as mentioned above. The polyethylene glycol with molecular weight of 600 is easy to melt when inserting the tampon 10 into the vaginal cavity, which makes the agent M more rapid-acting. In this point, the polyethylene glycol with molecular weight of 600 is superior.

Further, in the foregoing embodiment, as a manufacturing process for the tampon body 20, a method is provided in which the melted agent M is applied onto the outer surface 21 of the tampon body 20 after obtaining the tampon body 20 by compressing and shaping the absorbent-body material 60. However, the invention is not limited thereto. For example, it is also possible that after the agent M is applied to the cover 64, the absorbent main body 62 is covered with the cover 64 to form the absorbent-body material 60, and thereafter the absorbent-body material 60 is compressed and shaped to obtain the tampon body 20. Further, it is possible that after the absorbent main body 62 is covered with the cover 64 to form the absorbent-body material 60, the agent M is applied to the absorbent-body material 60 and the absorbent-body material 60 to which the agent M is applied is compressed and shaped to obtain the tampon body 20.

### Reference Signs List

10 tampon, 20 tampon body (absorbent body), 21 outer surface,
22 withdrawal string, 22a exposed portion, 23 applied portion, 25 non-applied portion,
30 applicator, 40 outer tube, 40a inner surface, 41 radially-large portion,
42 radially-small portion, 43 front-end opening, 44 petal portion, 45 rear-end opening,
46 annular rib, 47 shoulder, 50 inner tube, 51 first inner tube,
51a sword-guard portion, 51b annular projection, 52 second inner tube, 52a sword-guard portion,
52b projection section, 52c flared portion, 54 longitudinal rib,
60 absorbent-body material, 62 absorbent main body, 64 cover,
100 manufacturing apparatus, 102 compressing-shaping drum,
102a holding section,
104 heating drum, 104a holding section,
106 cooling drum, 106a holding section,
108 conveyor unit, 108a conveyor belt,
110 applying unit, 110a supplying unit, 110b transferring belt,
112 cool-air-blowing unit,
C center, E1 foremost end, E2 rearmost end, M agent.

## Claims

1. A tampon having an absorbent body that absorbs liquid, comprising:
on an outer surface of the absorbent body,
an applied portion to which an agent is applied,
the agent being a mixture of
an active pharmaceutical ingredient,
a first water-soluble carrier that carries the active pharmaceutical ingredient and is a main ingredient of the agent, and
a second water-soluble carrier that carries the active pharmaceutical ingredient and has a melting point lower than that of the first water-soluble carrier.

2. A tampon according to claim 1, wherein
the absorbent body includes, on the outer surface thereof, the applied portion to which the agent is applied and a non-applied portion to which the agent is not applied, and
by the applied portion and the non-applied portion, a pattern is formed on the outer surface.

3. A tampon according to claim 1 or 2, wherein
the first water-soluble carrier has a melting point higher than body temperature,
the second water-soluble carrier has a melting point lower than or equal to body temperature.
